# EUROPÄISCHE PATENTSCHRIFT

(11) **EP 0 306 874 B1**
(45) Veröffentlichungstag und Bekanntmachung des Hinweises auf die Patenterteilung: **14.12.1994**
(21) Anmeldenummer: 88114431.5
(22) Anmeldetag: 03.09.1988
(51) Int. Cl.: C07D 201/16

(54) **Verfahren zur Aufarbeitung von Destillationsrückständen, die bei der Reinigung von Caprolactam anfallen**
Process for working-up distillation residues obtained during the purification of caprolactam
Procédé d'élaboration de résidus de distillation obtenus lors de la purification du caprolactame

(30) Priorität: 05.09.1987 DE 3729853
(43) Veröffentlichungstag der Anmeldung: 15.03.1989
(73) Patentinhaber: BASF Aktiengesellschaft, 67063 Ludwigshafen (DE)
(72) Erfinder: Fuchs, Hugo, Dr., D-6700 Ludwigshafen (DE)

(56) Entgegenhaltungen:
- EP-A- 0 022 161
- EP-A- 0 138 241
- DD-A- 202 870
- DE-C- 858 702
- NL-A- 7 312 718
- CHEMICAL ABSTRACTS, Band 103, Nr. 10, 9. September 1985, Seite 7,Zusammenfassung Nr. 71812x, Columbus, Ohio, US;& NL-A-83 03 028 (STAMICARBON B.V.) 18-03-1985
- CHEMICAL ABSTRACTS, Band 92, Nr. 2, 14. Januar 1980, Seite 16, Zusammenfassung Nr. 7163p, Columbus, Ohio, US; & RO-A-62 566 (INTREPRINDERA DE FIBRE SINTETICE , SAVINESTI) 25-08-1973

## Beschreibung

Caprolactam wird bei dessen Herstellung zum Zwecke der Reinigung durch Destillation von leichtsiedenden und schwersiedenden Produkten getrennt. Die hierbei anfallenden hochsiedenden Destillationsrückstände enthalten noch Caprolactam dessen Oligomere und Polymere sowie nicht bestimmbare Verunreinigungen und Zersetzungsprodukte sowie gegebenenfalls Natriumhydroxyd, sofern man dieses vor der Destillation zugesetzt hat. Da diese Rückstände noch erhebliche Mengen Caprolactam enthalten, ist es angezeigt, dieses aus dem Rückstand zu gewinnen und zu verwerten.

Aus der japanischen Patentveröffentlichung 11332/66 ist bekannt, daß man den alkalischen Destillationsrückstand mit rauchender Schwefelsäure behandelt. Aufgrund der Salzbildung mit den in den Rückständen enthaltenen Alkalien kommt es zu Verkrustungen beim Destillieren, die zu Störungen Anlaß geben. Nach einem anderen in der EP-Anmeldung 22161 beschriebenen Verfahren wird aus dem alkalischen Destillationsrückstand in einer ersten Stufe bei einer Sumpftemperatur von 130 bis 160°C unter vermindertem Druck zunächst Caprolactam abdestilliert und aus dem nunmehr verbleibenden Rückstand einer zweiten Stufe bei einer Sumpftemperatur von 140 bis 180°C unter vermindertem Druck weiteres Caprolactam abdestilliert, das in einer dritten Stufe mit Säuren behandelt wird und wiederum in die Reinigungsoperation für das Caprolactam aus der Beckmannischen Umlagerung zurückgeführt wird. Ein solches Verfahren ist technisch aufwendig und führt immer noch zu Rückständen die schwer handhabbar sind.

In der DE-C 858702 wird ein Verfahren zum Reinigen von Caprolactam beschrieben, wobei Caprolactam bei erhöhter Temperatur mit einem Inertgas, gegebenenfalls in Gegenwart geringer Mengen alkalischer oder saurer Stoffe, behandelt wird.

Als Inertgas können gegen Caprolactam unter den Arbeitsbedingungen indifferenten Gase, z.B. Luft, Stickstoff, Wasserstoff und gasförmige Kohlenwasserstoffe verwendet werden.

Es war deshalb die technische Aufgabe gestellt aus Destillationsrückständen die bei der Reinigung von Caprolactam anfallen, Caprolactam möglichst vollständig auch aus den Oligomeren und Polymeren zurückzugewinnen.

Diese technische Aufgabe wird gelöst in einem Verfahren zur Aufarbeitung von Destillationsrückständen, die bei der Reinigung von Caprolactam anfallen, wobei man die Destillationsrückstände in Gegenwart von Natrium- oder Kaliumhydroxyd und in Gegenwart eines hochsiedenden Kohlenwasserstoffs auf eine Temperatur von 250 bis 500°C erhitzt und Caprolactam fortlaufend aus dem Reaktionsgemisch entfernt.

Das neue Verfahren hat den Vorteil, daß man Caprolactam weitgehend aus den Destillationsrückstänen zurückgewinnt in einer Form, die es gestattet, dieses wiederum in da Aufarbeitungsverfahren zurückzuführen ohne daß nachteilige Folgen eintreten.

Erfindungsgemäß geht man von Destillationsrückständen, die bei der Reinigung von Caprolactam anfallen aus. Solche Destillationsrückstände erhält man beispielsweise nach Abtrennen von Caprolactam bei Sumpftemperaturen von 120 bis 150°C unter vermindertem Druck, z. B. 1,3 bis 20 mbar. Die Destillationsrückstände enthalten neben Caprolactam, Oligomere des Caprolactams, Polymere des Caprolactams sowie nicht näher definierte Zersetzung und Umsetzungsprodukte sowie gegebenenfalls Natriumhydroxyd. Ein geeigneter Destillationsrückstand fällt z. B. nach dem in der EP-Anmeldung 22161 beschriebenen Verfahren an.

Die Behandlung wird in Gegenwart von Natrium- oder Kaliumhydroxyd durchgeführt. Vorteilhaft wendet man je Gewichtsteil Destillationsrückstand 2 bis 10, insbesondere 4 bis 8 Gew.-Teile Natrium- oder Kaliumhydroxyd an. Falls der Destillationsrückstand bereits Natriumhydroxyd enthält, wird die Menge entsprechend ergänzt.

Darüber hinaus wird die Behandlung der Destillationsrückstände in Gegenwart von hochsiedenden Kohlenwasserstoryffen durchgeführt. Die verwendeten Kohlenwasserstoffe haben einen Siedepunkt über 250°C, insbesondere von 350 bis 450°C. Zweckmäßig liegt der Siedepunkt der verwendeten Kohlenwasserstoffe über demjenigen des Caprolactams. Geeignete Kohlenwasserstoffe sind hochsiedende Mineralöle, Gasöl, Vaxuumgasöl, Heizöl, technisches Weißöl, geschmolzenes Paraffinwachs oder aromatisches Kohlenwasserstofföl. Vorzugsweise verwendet man je Gewichtsteil Destillationsrückstand 0,1 bis 10 Gew.-Teile, insbesondere 1 bis 5 Gew.-Teile hochsiedende Kohlenwasserstoffe an.

Die Behandlung wird bei einer Temperatur von 250 bis 450°C, insbesondere von 280 bis 380°C durchgeführt. In der Regel wendet man Atmosphärendruck oder verminderten Druck, z. B. von 20 bis 200 mbar an.

Ein wesentliches Merkmal der Erfindung ist es, daß Caprolactam fortlaufend aus dem Reaktionsgemisch entfernt wird. Um dies zu bewirken, hat es sich bewährt, wenn man Inertgase durch das Reaktionsgemisch leitet. Geeignete Inertgase sind z. B. Stickstoff, Kohlendioxid, Rauchgase oder überhitzter Wasserdampf. Besonders geeignet ist Stickstoff. Zweckmäßig wendet man je kg/Reaktionsgemisch 1 bis 100 Nl/Std. Inertgase an. Andererseits ist es auch möglich, Caprolactam durch Anwendung von vermindertem Druck, wie oben ausgeführt, allein oder durch zusätzliche Mitverwendung von Inertgasen fortlaufend abzutrennen. Das aus dem Reaktionsgemisch austretende Caprolactam wird zweckmäßig durch Kühlen kondensiert oder wird durch Waschen mit einem geeigneten Lösungsmittel, wie Wasser abgeschieden. Sofern Inertgase mitverwendet werden, können diese zweckmäßig zurückgeführt werden.

Das so zurückgewonnene Caprolactam setzt man vorteilhaft wiederum dem Rohlactam zu, das bei der Beckmannschen Umlagerung nach dessen Neutralisation anfällt. Ebenfalls ist es möglich, das jeweils gewonnene Caprolactam während der Neutralisation dem Umlagerungsgemisch zuzuführen.

Die mitverwendeten hochsiedenden Kohlenwasserstoffe können mehrfach verwendet werden. Zweckmäßig werden die hochsiedenden Kohlenwasserstoffe gereinigt, z. B. durch Filtration oder Zentrifugieren, ferner durch Waschen mit Wasser oder verdünnten Säuren. Sofern wenig aufwendige hochsiedende Kohlenwasserstoffe verwendet werden, führt man sie der Entsorgung durch Unterfeuerung zu.

Das Verfahren nach der Erfindung sei an folgenden Beispielen veranschaulicht. Die angegebenen Teile sind Gewichtsteile, sie verhalten sich zu den Volumenteilen wie kg zu l.

### Beispiel 1

In einem Reaktionsgefäß werden 100 Teile eines Destillationsrückstandes aus der Caprolactamherstellung mit einem Restlactamgehalt von 3 Gew.% und einem Gehalt von 5,8 Gew.% Natriumhydroxid mit 200 Teilen technischem Weißöl vermischt und auf 380°C erhitzt. Gleichzeitig werden stündlich 10 000 Volumenteile Stickstoff in die Mischung eingeleitet. Aus den austretenden Gasen wird durch Kühlen Caprolactam kondensiert. Innerhalb von 1,8 Stunden werden 56,5 Teile Caprolactam erhalten.

### Beispiel 2

Wie in Beispiel 1 beschrieben, werden 100 Teile eines Destillationsrückstandes mit einem Restlactamgehalt von 3 Gew.% und einem Gehalt von 5,8 Gew.% Natriumhydroxid mit 200 Teilen technischem Weißöl vermischt und auf 330°C erhitzt. Der Druck beträgt 40 bis 60 mbar, innerhalb 1 Stunde werden als Destillat 60 Teile Caprolactam erhalten.

## Patentansprüche

1. Verfahren zur Aufarbeitung von Destillationsrückständen, die bei der Reinigung von Caprolactam anfallen, dadurch gekennzeichnet, daß man die Destillationsrückstände, die neben Caprolactam Oligomere des Caprolactams, Polymere des Caprolactams sowie nicht näher definierte Zersetzungs- und Umsetzungsprodukte sowie ggf. Natriumhydroxid enthalten, in Gegenwart von Natrium- oder Kaliumhydroxid und in Gegenwart eines über 250°C siedenden Kohlenwasserstoffs auf eine Temperatur von 250 bis 500°C erhitzt und Caprolactam fortlaufend aus dem Reaktionsgemisch entfernt.

2. Verfahren nach Anspruch 1, dadurch gekennzeichnet, daß man je Gewichtsteil Destillationsrückstand 2 bis 10 Gew.% Natrium- oder Kaliumhydroxid anwendet.

3. Verfahren nach den Ansprüchen 1 und 2, dadurch gekennzeichnet, daß man je Gewichtsteil Destillationsrückstand 0,1 bis 10 Gew.-Teile hochsiedende Kohlenwasserstoffe mitverwendet.

4. Verfahren nach den Ansprüchen 1 bis 3, dadurch gekennzeichnet, daß man Kohlenwasserstoffe mit einem Siedepunkt der über demjenigen des Caprolactam liegt, verwendet.

5. Verfahren nach den Ansprüchen 1 bis 4, dadurch gekennzeichnet, daß man eine Temperatur von 280 bis 380°C einhält.

6. Verfahren nach den Ansprüchen 1 bis 5, dadurch gekennzeichnet, daß man verminderten Druck anwendet.

7. Verfahren nach den Ansprüchen 1 bis 6, dadurch gekennzeichnet, daß man Caprolactam durch Zuführen von Inertgas fortwährend aus dem Reaktionsgemisch abtrennt.

## Claims

1. A process for working up distillation residues from the purification of caprolactam, characterized in that the distillation residues, which besides caprolactam contain oligomers of caprolactam, polymers of caprolactam and unspecified decomposition and reaction products with or without sodium hydroxide, are heated in the presence of sodium hydroxide or potassium hydroxide and in the presence of a hydrocarbon having a boiling point above 250°C to a temperature from 250 to 500°C and caprolactam is continuously removed from the reaction mixture.

2. A process as claimed in claim 1, characterized in that from 2 to 10% by weight of sodium hydroxide or potassium hydroxide is employed per part by weight of distillation residue.

3. A process as claimed in claim 1 or 2, characterized in that from 0.1 to 10 parts by weight of high-boiling hydrocarbon are used per part by weight of distillation residue.

4. A process as claimed in any of claims 1 to 3, characterized in that a hydrocarbon having a boiling point above that of caprolactam is used.

5. A process as claimed in any of claims 1 to 4, characterized in that a temperature from 280 to 380°C is maintained.

6. A process as claimed in any of claims 1 to 5, characterized in that reduced pressure is employed.

7. A process as claimed in any of claims 1 to 6, characterized in that caprolactam is continuously removed from the reaction mixture by infeeding an inert gas.

## Revendications

1. Procédé de traitent ultérieur de résidus de distillation qui sont formés au cours de la purification de caprolactame, caractérise en ce qu'on chauffe à une température de 250 à 500°C, en présence d'hydroxyde de sodium ou de potassium et en présence d'un hydrocarbure de point d'ébullition supérieur à 250°C, les résidus de distillation qui contiennent, en dehors de caprolactame, des oligomères du caprolactame, des polymères du caprolactame, ainsi que des produits de décomposition et de réaction non définis plus précisément et éventuellement de l'hydroxyde de sodium, et on élimine le caprolactame de façon continue du mélange réactionnel.

2. Procédé selon la revendication 1, caractérisé en ce qu'on utilise, par partie en poids de résidu de distillation, de 2 à 10% en poids d'hydroxyde de sodium ou de potassium.

3. Procède selon la revendication 1 ou 2, caractérisé en ce qu'on utilise simultanément, par partie en poids de residu de distillation, de 0,1 à 10 parties en poids d'hydrocarbures de point d'ébullition élevé,

4. Procédé selon l'une quelconque des revendications 1 à 3, caractérisé en ce qu'on utilise des hydrocarbures ayant un point d'ébullition qui se situe au-dessus de celui du caprolactame.

5. Procédé selon l'une quelconque des revendications 1 à 4, caractérisé en ce qu'on maintient une température de 280 à 380°C.

6. Procédé selon l'une quelconque des revendications 1 à 5, caractérisé en ce qu'on applique une pression réduite.

7. Procédé selon l'une quelconque des revendications 1 à 6, caractérisé en ce qu'on sépare le caprolactame de façon continue du mélange réactionnel, par apport de gaz inerte.
